# EUROPEAN PATENT APPLICATION

(11) **EP 2 184 100 A1**
(43) Date of publication of application: **12.05.2010**
(21) Application number: 08783740.7
(22) Date of filing: 25.07.2008
(51) Int. Cl.: B01F 17/00, A61K 9/107

(54) **A COMPOSITE EMULSIFIER, AN EMULSION PREPARED FROM IT AND THE PREPARATION METHOD THEREOF**

(30) Priority: 26.07.2007 CN 200710012275
(71) Applicant: Shenyang Pharmaceutical University, Shenyang Liaoning 110016 (CN); Wenzhou Haijiang Pharmaceutical Technology Co., Ltd, Zhejiang 325000 (CN)
(72) Inventor: DENG, Yihui, Shenyang Liaoning 110016 (CN); ZHAO, Jing, Shenyang Liaoning 110016 (CN); DONG, Xiaohui, Shenyang Liaoning 110016 (CN); SHI, Li, Shenyang Liaoning 110016 (CN); LU, Yi, Shenyang Liaoning 110016 (CN); NI, Dongmin, Shenyang Liaoning 110016 (CN); ZHAO, Hongwei, Shenyang Liaoning 110016 (CN)
(74) Representative: Crowhurst, Charlotte Waveney
(86) International application number: PCT/CN2008/071747
(87) International publication number: WO 2009/012718

(57) **Abstract**

A composite emulsifier, contains two or more emulsifiers selected from phospholipid, PEG emulsifier and poloxamer-like substance, and may contain cryoprotectant An emulsion prepared from the composite emulsifier and a preparation method of the emulsion.

## Description

### Field of the Invention

The present application relates to the field of pharmaceuticals, specifically relates to a composite emulsifier, an emulsion prepared from the composite emulsifier and the preparation method thereof.

### Background of the invention

Generally, an emulsion comprises "oil phase", "water phase" and "emulsifier". The conventional (typical or traditional) emulsions are very susceptible to environmental conditions due to their unstability. For instance, some operations, such as freezing, shaking and sterilizing at high temperatures, will result in the alteration of particle size, or even demulsification and oil separation, which may observably change the quality of the product, affect the therapeutic effects, bring unexpected adverse reactions, or even cause useless products and great loss. Currently, oil phases for preparing emulsions mostly utilize long-chain fatty acid esters, such as soybean oil. Long-chain fatty acid esters have many disadvantages. For example, soybean oil has long carbon chains, high viscosity, and low solubility for lipophilic drugs; and further has a large amount of unsaturated double bonds, and thus is susceptible to oxidization. More seriously, long-chain fatty acid esters, even medium-chain triglyceride (MCT) or the mixtures of long-chain triglyceride and MCT, are unendurable to freeze-thaw process, and will greatly increase the particle size during the sterilization at high temperatures. This is mainly because only one or two emulsifiers are used in the formulations of the current emulsions. When the combinations (unoptimized) of two emulsifiers are used, the elements to be concerned are merely no demulsification occurred during high-temperature sterilization and no great increase of the particle size after sterilization, but without the resistance to both high-temperature sterilization and freeze-thaw process to be taken into consideration. Emulsions have been studied from 1678, and have received wide attention since Intralipid was launched successfully in 1961. Emulsions have gained great success and made an important contribution to human life and health. However, it is regrettable that the classical prescription (mainly comprising 10% (or 20% or 30%) soybean oil, 1.2% lecithin for injection; 2.2% glycerol, pH = ~8) utilized years ago (1961) is still being used with a little alteration, if any, while the advanced techniques have been incorporated into the manufacturing process and equipments. For generating more negative charges and stabilizing the emulsion, an appropriate amount of oleic acid is often added into above mentioned formulations and simultaneously the pH value is adjusted to about 9, so as to obtain a pH of about 8 after sterilization. Those formulations and process for emulsions have the disadvantages of: 1) the emulsions thus prepared are unendurable to freeze-thaw process; 2) oleic acid added into the emulsions is hemolytic in nature; 3) filtration-sterilization is infeasible due to the large average particle size, and the complexity and cost of the process are thus increased by utilization of rotation-sterilization; 4) the high pH value is not suitable for the drugs that are unstable under high pH (such as diisopropylphenol and alprostadil); and 5) the emulsion thus prepared should be stored in a refrigerator at low temperatures. With the development of the techniques, there is a need to improve the classical emulsion formulation so as to solve the problems mentioned above.

For improving the freeze-thaw resistance of the emulsions, the most common way is to reasonably select the components of emulsifiers and oil phases. It has been reported that, when tween 80 or polyoxyethylenated castor oil is used for preparing emulsions, they exhibit some protective effects for the emulsions. However, such protective effects are far from satisfactory. The particle size of the emulsion thus obtained is too large to be sterilized via filtration. Moreover, the potent histamine-releasing effects may induce allergy in patients, or even death due to the allergy

The background knowledge of the lipophilic drugs will be described hereinafter with coenzyme Q₁₀ as an example.

Coenzyme Q₁₀ is an important member in the family of coenzyme Q, which widely exists in the inner mitochondrial membrane, and plays important roles in human cells. The total amount of endogenous coenzyme Q₁₀ in human body is 0.5-1.5 g with higher levels in heart, liver and pancreas. Coenzyme Q₁₀ is partly synthesized in human body, and the rest are taken from food. The synthesis of coenzyme Q₁₀ in human body decreases gradually with age.

Coenzyme Q₁₀ exhibits anti-oxidative, free radical-scavenging, biomembrane-stabilizing and immunity-improving effects, and is used in clinic for treating and/or preventing cardiovascular diseases, hepatic diseases (such as acute and chronic hepatitis and subacute hepatic necrosis), cancers, degeneration and disorder of central nervous systems, etc. Coenzyme Q₁₀ is also used for preventing or reducing the side effects of some drugs, such as statins or cell inhibitors, for example the cardiotoxicity of adriamycin.

Recently, a number of studies have been carried out on the extraction and manufacture of coenzyme Q₁₀. Japan as the first country exploiting coenzyme Q₁₀, becomes the largest production country in the world. According to the statistics, 90% of the coenzyme Q₁₀ comes from Japan. Currently, some enterprises in China have gradually grasped the advanced techniques, including bio-extraction method, semi-synthesis method and microbial fermentation method, etc., and thus provide a basis for the domestic need of coenzyme Q₁₀. The marketed dosage forms of coenzyme Q₁₀ mainly include tables, capsules, soft capsules, injections, etc, but still could not meet the demands for the treatment of diseases. Coenzyme Q₁₀ is a lipophilic substance with very poor solubility in water (almost insoluble in water). The tablets and capsules for oral administration have the disadvantages of low bioavailability, large individual differences, etc., while the injections exhibit poor physical stability and have tendency to cause precipitation of drug during storage, and thus require heating for re-dissolving. Tween 80 is usually added as a solubilizing agent for increasing the solubility of coenzyme Q₁₀. However, tween 80 has hemolytic effects, and is susceptive to oxidization. The oxidative products of tween 80 may lead to allergy.

If coenzyme Q₁₀ is manufactured into emulsions, the solubility, bioavailability and targeting ability thereof may be increased, while the toxicity and side effects may be reduced.

China patent ZL02808179.X discloses a coenzyme Q₁₀-containing micro-emulsion pre-concentrate and a micro-emulsion mainly used for oral administration. China application CN 200480017624.9 discloses a chemical composition for increasing delivery and absorption of coenzyme Q₁₀ by using essential oil(s) and the method of preparation thereof. CN 200610046134.2 discloses coenzyme Q₁₀ emulsion injections and the preparation method thereof.

However, the emulsions prepared according to the examples 2 and 3 of CN 200610046134.2 produce precipitates drug after standing for 2h and 1 h, respectively. As for the other formulations, no better result is reached according to experiments. The emulsions prepared in accordance with conventional methods do not have the freeze-thaw resistance, either.

The inventor tried to prepare coenzyme Q₁₀ emulsions in accordance with conventional methods by using single emulsifier of lecithin (2%) or Solutol HS₁₅ (2%) as the emulsifier, and measure the thermo-resistance and freeze-thaw stability of the emulsions thus obtained. The results are shown in Table 1.

**Table 1: Thermo-resistance and freeze-thaw stability of the emulsions prepared with single emulsifier**

| | Lecithin | | | Solutol HS₁₅ | | |
|---|---|---|---|---|---|---|
| | Average particle size (nm) | 99% particle size (nm) | CV | Average particle size (nm) | 99% particle size (nm) | CV |
| Initiation | 115.8 | 251.6 | 0.362 | 67.1 | 163.7 | 0.422 |
| Moist heat sterilization | 111.9 | 246.9 | 0.370 | phase separation | | |
| Freeze-thaw experiments | 265.7 | 448.1 | 0.337 | 68.8 | 167.6 | 0.421 |

It can be seen from Table 1 that the emulsions with phospholipid or HS₁₅ had good centrifugation stability No change of average particle size and particle size distribution was observed before and after centrifugation. The emulsion with phospholipid alone as the emulsifier had good sterilization stability, but the particle size thereof increased after freeze-thaw. The emulsion with HS₁₅ alone as the emulsifier was endurable to freeze-thaw but unendurable to sterilization.

For improving both thermo-resistance and freeze-thaw resistance of the emulsions, in the first aspect, the present invention provides a composite emulsifier

In the second aspect, the present invention provides an emulsion prepared with the composite emulsifier.

In the third aspect, the present invention provides a method for preparing the emulsion.

### Summary of the Invention

The composite emulsifier of the present invention comprises two or more of the following materials: a phospholipid ≤10%w/v, a PEG emulsifier ≤30%w/v, a poloxamer ≤10%w/v, calculated based on the emulsion.

For achieving better freeze-thaw resistance, the composite emulsifier of the present invention may further comprise a cryoprotectant in an amount of ≤50%w/v.

The phospholipid used in the composite emulsifier of the present invention may be selected from one or more of yolk phospholipids, soybean phospholipids and other natural, semi-synthesized or synthesized phospholipids. The poloxamer used may be selected from the group consisting of Poloxamer188 and Pluronic F68. The PEG emulsifier may be selected from one or more of polyethylene glycol 12-hydroxystearate (polyethylene glycol 660 hydroxystearate, Solutol, HS₁₅), tocopherol polyethylene glycol succinate (TPGS) and DSPE-PEG (including DPPE-PEG, DMPE-PEG), wherein the molecular weight of PEG is 100-10000. The useful cryoprotectant may be selected from the group consisting of alcohols and saccharides.

In a preferred embodiment, the composite emulsifier consists of a phospholipid, a PEG emulsifier and a cryoprotectant.

The composite emulsifier of the present invention may comprise three or more emulsifiers, one of which is poloxamer, but does not comprise a cryoprotectant

The inventor found that freeze-thaw resistance of an emulsion can be achieved without a cryoprotectant by using a composite emulsifier comprising three or more emulsifiers, and one of which is poloxamer. The emulsion can include any of medium-chain triglyceride (MCT), long-chain triglyceride (LCT), a mixture of MCT and LCT, an oily material from traditional Chinese medicines, and a lipophilic compound dissolved in oil used as the oil phase. In a preferred embodiment, the composite emulsifier consists of one or two of a PEG emulsifier (including HS₁₅, TPGS, DSPE-PEG2000) and a phospholipid, as well as a poloxamer emulsifier. The cryoprotectant in the composite emulsifier is not necessarily added. The composite emulsifier is widely applicable to various oil phases, including C₆-C₂₈ oils and lipophilic compounds dissolvable in these oils. The composite emulsifier can also be used in an oil for therapeutic use or a lipophilic compound dissolved in the oil for therapeutic use to obtain a preparation.

In the second aspect, the present invention provides an emulsion prepared with the composite emulsifier of the invention. The emulsion comprises an oil phase, the composite emulsifier and a water phase, wherein the composite emulsifier comprises two or more of the following materials: a phospholipid ≤10%w/v, a PEG emulsifier ≤30%w/v, and a poloxamer ≤10%w/v, calculated based on the emulsion.

The oil phase of the present emulsion comprises a C₆-C₂₈ oil, an oily material with therapeutic activity and/or a lipophilic compound or drug dissolved or dispersed in the C₆-C₂₈ oil. The weight ratio of the lipophilic compound or drug to the C₆-C₂₈ oil is 1:0 to 1:10000 w/w. The amount of the C₆-C₂₈ oil is 0.1-20% (w/v), calculated based on the emulsion.

The C₆-C₂₈ oil may be selected from one or more of structurally modified or hydrolyzed coconut oil, olive oil, soybean oil, safflower oil, triglycerides, octyl and decyl glycerate, ethyl oleate, glyceryl linoleate, ethyl linoleate, glyceryl oleate, cholesteryl oleate/linoleate, coconut oil C₈/C₁₀ monoglyceride or diglyceride, coconut oil C₈/C₁₀ propanediol diester, and coconut oil C₈/G₁₀ triglycerides.

The triglycerides above in the C₆-C₂₈ oil may be medium-chain triglycerides (MCT) and/or long-chain triglycerides (LCT). MCT has the advantages of short carbon chains, low viscosity, high solubility for lipophilic drugs, oxidization resistance due to the absence of double bonds, and simple metabolism *in vivo*, as compared with long-chain fatty acid esters, such as soybean oil. The purpose of the present invention can be achieved by using MCT and LCT in combination to obtain an appropriate viscosity of oil phase, which is emulsified with the composite emulsifier (consisting of one or more of phospholipids and emulsifiers containing PEG chains, such as HS₁₅, TPGS, DSPE-PEG (the molecular weigh of PEG = 100-10000)) and adding a cyoprotectant.

The oily material with therapeutic activity and/or the lipophilic compound or drug dissolved or dispersed in the C₆-C₂₈ oil is selected from the group consisting of coenzyme Q₁₀, cucurbitacin, cucurbitacin B, dihydrocucurbitacin B, isocucurbitacin B, cucurbitacin D, cucurbitacin E, cucurbitacin I, cucurbitacin Q, alprostadil, diisopropylphenol, vitamin K1, dexamethasone palmitate, tanshinone IIA, butylphthalide, ligustilide, irisquinone, entecavir, anethol trithione, malotilate, homoharringtonine, demethylcantharidate, curcumine, cyclandelate, β-elemene, batyl alcohol, the hypolipidemic drug of statins (such as lovastatin and simvastatin, etc.), *Brucea javanica* oil, sea buckthom oil, fish oil (deep see fish oil, rich in polyunsaturated fatty acids and esters thereof, such as DHA), coix seed oil , zedoary turmeric oil, garlic oil, allicin, *Chuanxiong* rhizome oil, angelica oil, capsicum oil, ginger oil, celery seed oil, *Houttuynia cordata* oil, Evening primrose oil, perilla seed oil, Wufu Xinnaokang (also known as Wufu Xinnaoqing, Xinnaoqing/Xinnaokang, mainly comprising refined safflower oil, vitamin B₆, vitamin E, borneol, etc.), tocopherol nicotinate, gossypol, oridonin, fenofibrate, itraconazole, candesartan, hydroxycamptothecin, camptothecin derivatives, paclitaxel and the derivatives thereof, docetaxel and the derivatives thereof, finasteride, etoposide, ginsenoside, 20(S)-protopanaxadiol, ginsenoside Re, ginsenoside Rb1, ginsenoside Rg₂, 20(R)-ginsenoside Rg₃ and 20(S)-ginsenoside Rg₃.

The emulsion of the present invention may also comprise one or more pharmaceutically acceptable excipients selected from a co-emulsifier, a stabilizer, a cryoprotectant and a pH adjuster.

The useful co-emulsifier in the emulsion of the present invention is selected from one or more of oleic acid, linoleic oil, linolenic acid, stearic acid, docosahexaenoic acid and cholic acid. The useful cryoprotectant is selected from one or more of alcohols (including propanediol, glycerol and polyethylene glycol), and one or more of saccharides (including glucose, mannitol, sucrose, trehalose, xylitol, maltose and lactose). The useful stabilizer is selected from one or more of nitrogen, EDTA and salts thereof, anhydrous sodium sulfite, anhydrous sodium hydrogen sulfite, sodium pyrosulfite, vitamin C and derivatives thereof, butylated hydroxytoluene, α-tocopherol, α-tocopherol acetate and hydroquinone. The useful isoosmotic adjustment agent is selected from one or more of glycerol, 1,2-propanediol, glucose, maltose, mannitol and xylitol. The useful pH adjuster is selected from one or more of hydrochloric acid, sodium hydroxide, acetic acid, sodium acetate, phosphoric acid, sodium phosphate, citric acid and sodium citrate.

The invention also provides a method for preparing the emulsion with the composite emulsifier of the invention, comprising the steps of:
1) preparing the oil phase: heating the oil and lipophilic material to 20-90°C under nitrogen atmosphere, then adding the emulsifier, stirring at 20-90°C till dissolved, and adding an active ingredient under stirring;
2) preparing the water phase: adding a water-soluble material (isoosmotic adjustment agent, cryoprotectant, etc.) into water or a emulsifier used in water phase, and stirring at 20-90°C for 5 min till completely mixed and dissolved;
3) adding the water phase into the oil phase under nitrogen atmosphere at 20-60°C, and stirring for 5-30 min to obtain the primary emulsion;
4) homogenizing with a homogenizer firstly under a pressure of 4000-8000 psi and then under a pressure of 10000-30000 psi to obtain the emulsion.

The pH value of the primary emulsion may be adjusted to 3-9 before the step of homogenizing, if necessary.

Emulsions for injection can be obtained by subjecting the above emulsion to filtration with microporous membrane, package and sterilization. The sterilization can be selected from moist heat sterilization, filtration sterilization or microwave sterilization.

The emulsion according to this invention has average particle size of less than 150 nm, more preferably, less than 100 nm. This emulsion is convenient for parenteral, oral or topical administration.

Comparing with the prior arts, the present invention has the advantages of:
1) the emulsion thus prepared can resist the destructive effects of heat and freeze-thaw; 2) it is easier to produce emulsions with average particle size of less than 150 nm, or even less than 100 nm; and has low requirement for equipment; 3) filtration sterilization can be utilized to obtain a sterile preparation without high temperatures; 4) the emulsion is little affected by pH and other additives in the formulation; the emulsion can be manufactured at low pH, and thus have wider usage; 5) the drugs in the form of emulsion have improved chemical stability; 6) the composite emulsion layer existing on the surface can greatly reduce the stimulation of the drugs.

### Description of the Drawings

Figure 1 is the electrocardiogram of the rat before adriamycin treatment in Example 16.
Figure 2 is the electrocardiogram of the rat after adriamycin treatment in Example 16.
Figure 3 is the electrocardiogram of the rat after administrating coenzyme Q₁₀ emulsion in Example 16.

### Detailed description of the Invention

The invention will be described in details with reference to the following examples. It should be understood, the following examples are used for illustrating the invention but not intended to limit the invention to these examples. The present invention encompasses various modifications and/or alterations as will be appreciated by those skilled in the art.

In the following examples, the content of coenzyme Q₁₀ was detected via HPLC chromatography using C₁₈ alkyl-silane bonded silica gel chromatography column with methanol-ethanol (50/50, v/v) as the mobile phase at a detection wavelength of 275 nm. PSS.NICOMP^{™} 380 was used as the instrument for detecting the particle size of the emulsions.

### Example 1: Effects of oil phases with different soybean oivmedium-chain oil ratios on the particle sizes of the emulsions before and after freeze-thaw, when HS₁₅ and SPC (soybean lecithin) are used as the composite emulsifier

Formulation (100 mL): coenzyme Q₁₀ 0.25 g, SPC 1.2 g, HS₁₅ 0.9 g, injectable oils 10 g, glycerol 2.2 g, and injectable water as the balance. The injectable oils were soybean oil and medium-chain oil with the ratio of 100:0 to 0:100 (w/w).

Preparation process: 1) heating the injectable oils, SPC and HS₁₅ in a preparation tank till 50°C, strongly stirring till dissolved, and adding the drug, stirring till dissolved (adding active carbon, if necessary; removing the pyrogens by conventional methods); 2) adding glycerol into injectable water in another preparation tank, stirring at 50°C for 5 min till dissolved (adding active carbon, if necessary; and removing the pyrogens by conventional methods); 3) adding the product coming from step 2) into the product coming from step 1), stirring at 50°C to obtain the primary emulsion; 4) passing the primary emulsion (liquid) through a microfluidizer, adjusting the homogenizing pressure to 4000 psi at first, and then adjusting to 16000 psi, repeating the homogenization to obtain homogeneous emulsion. The coenzyme Q₁₀ emulsion for injection was obtained after filtration with membrane, package, filling with nitrogen, sealing and sterilization. The obtained emulsion was subjected to freeze-thaw experiments. The results are shown in Table 2.

**Table 2: Effects of different soybean oil/medium-chain oil ratios on particle size of emulsions before and after freeze-thaw**

| Soybean oil/ medium-chain oil | Original emulsion | After freeze-thaw | Increase of particle size |
|---|---|---|---|
| Weight ratio | Average particle size (nm) | Average particle size (nm) | Percent |
| 100/0 | 136.5 | 386.2 | Demulsification |
| 90/10 | 121.3 | 238.6 | Demulsification |
| 75/25 | 76.6 | 103.9 | Demulsification |
| 50/50 | 74.1 | 181.1 | 144.4% |
| 40/60 | 80.1 | 112.7 | 40.7% |
| 25/75 | 76.6 | 103.9 | 35.6% |
| 20/80 | 77.5 | 100.6 | 29.6% |
| 15/85 | 75.4 | 90.6 | 20.2% |
| 10/90 | 79.2 | 99.4 | 25.5% |
| 0/100 | 73.1 | 219.6 | 200.4% |

It can be seen from Table 2, when HS₁₅ and SPC are used as the composite emulsifier, the emulsion prepared at the soybean oil/medium-chain oil ratio in the range of 25:75-10:90 (w/w) exhibited less particle size increase after freeze-thaw. Thus, this ratio was selected as the preferred combination ratio.

### Example 2: Effects of oil phases with different soybean oil/medium-chain oil ratios on the particle sizes of the emulsions before and after freeze-thaw, when TPGS and SPC are used as the composite emulsifier

Formulation (100 mL): coenzyme Q₁₀ 0.25 g, SPC 1.2 g, TPGS 1 g, injectable oils 10 g, glycerol 2.2 g, and injectable water as the balance. The injectable oils are soybean oil and medium-chain oil with the ratio of 100:0 to 0:100 (w/w).

Preparation process: 1) heating the injectable oils, SPC and TPGS in a preparation tank till 50°C, strongly stirring till dissolved, and adding the drug, stirring till dissolved (adding active carbon, if necessary; removing the pyrogens by conventional methods); 2) adding glycerol into injectable water in another preparation tank, stirring at 50°C for 5 min till dissolved (adding active carbon, if necessary; and removing the pyrogens by conventional methods); 3) adding the product coming from step 2) into the product coming from step 1), stirring at 50°C to obtain the primary emulsion; 4) passing the primary emulsion (liquid) through a microfluidizer, adjusting the homogenizing pressure to 4000 psi at first, and then adjusting to 16000 psi, repeating the homogenization to obtain homogeneous emulsion. The coenzyme Q₁₀ emulsion for injection was obtained after filtration with membrane, package, filling with nitrogen, sealing and sterilization. The obtained emulsion was subjected to freeze-thaw experiments. The results are shown in Table 3.

**Table 3: Effects of different soybean oil/medium-chain oil ratios on particle sizes of emulsions before and after freeze-thaw**

| Soybean oil/ medium-chain oil | Original emulsion | After freeze-thaw | Increase of particle size |
|---|---|---|---|
| Weight ratio | Average particle size (nm) | Average particle size (nm) | Percent |
| 100/0 | 112 | 186 | 66% |
| 90/10 | 102 | 138 | 35% |
| 75/25 | 78 | 106 | 36% |
| 50/50 | 71 | 86 | 21% |
| 40/60 | 66 | 82 | 24% |
| 25/75 | 69 | 83 | 20% |
| 10/90 | 65 | 81 | 25% |
| 0/100 | 63 | 80 | 27% |

It can be seen from Table 3, when TPGS and SPC are used as the composite emulsifier, the emulsion prepared at the soybean oil/medium-chain oil ratio in the range of 100:0-0:100 (w/w) exhibited better freeze-thaw resistance than the emulsion prepared with HS₁₅. The increase of the particle size was smallest in the range of 50:50-0:100 (w/w).

When the soybean oil/medium-chain oil ratio is 0:100 (w/w), and the amount of TPGS in the formulation was changed to 5%, 10%, 15%, 20%, 25% or 30% (w/v) with other components unchanged, the emulsions thus obtained have the average particle sizes of 51 nm, 46 nm, 39 nm, 36 nm, 33 nm and 18 nm, respectively, and the particle size increased by less than 20%, suggesting good freeze-thaw resistance. Furthermore, the increased TPGS amount is in favor of improving the freeze-thaw resistance of the emulsions.

When the soybean oil/medium-chain oil ratio is 0:100 (w/w), and the amount of SPC in the formulation was changed to 2%, 5% or 10% (w/v) with other components unchanged, the emulsions thus obtained have the average particle sizes of 59 nm, 51 nm and 36 nm, respectively, and the particle size increased by less than 20%, suggesting good freeze-thaw resistance. Furthermore, the increased amount of SPC is in favor of improving the freeze-thaw resistance of the emulsions.

### Example 3: Effects of different phospholipids on particle sizes of the emulsions before and after freeze-thaw

Formulation (100 mL): coenzyme Q₁₀ 1.0 g, phospholipids 1.2 g, HS₁₅ 3.0g, injectable oils 10 g (soybean oil/medium-chain oil = 15/85 (w/w)), propanediol 5.0 g, EDTA-2Na 0.05 g and injectable water as the balance.

Preparation process: 1) heating the injectable oils, phospholipids and HS₁₅ in a preparation tank till 50°C, strongly stirring till dissolved, and adding the drug, stirring till dissolved (adding active carbon, if necessary; and removing the pyrogens by conventional methods); 2) adding propanediol and EDTA-2Na into injectable water in another preparation tank, stirring at 50°C for 5 min till completely dissolved (adding active carbon, if necessary; and removing the pyrogens by conventional methods); 3) adding the product coming from step 2) into the product coming from step 1), stirring at 50°C to obtain the primary emulsion; 4) passing the primary emulsion (liquid) through a microfluidizer, adjusting the homogenizing pressure to 5000 psi at first, and then adjusting to 14000 psi, repeating the homogenization to obtain homogeneous emulsion. The injectable coenzyme Q₁₀ emulsion was obtained after filtration with membrane, package, filling with nitrogen, sealing and sterilization. The obtained emulsion was subjected to freeze-thaw experiments. The results are shown in Table 4.

**Table 4: Effects of different phospholipids on freeze-thaw stability of coenzyme Q₁₀ emulsions**

| phospholipids types | Average particle size of original emulsion (nm) | Average particle size of emulsion after freeze-thaw (nm) |
|---|---|---|
| Domestic made soybean phospholipids (PC 80%) | 82.8 | 79.8 |
| Epikuron SPC 170 | 71.3 | 73.6 |
| Epikuron SPC 200 | 73.5 | 99.8 |
| Lipoid EPC | 70.2 | 72.7 |
| Lipoid S100 | 63.5 | 90.1 |

It can be seen from Table 4, the phospholipids types exhibited little effects on the particle size of the emulsions before and after freeze-thaw.

### Example 4: Effects of different cryoprotectants on the particle sizes of the emulsions before and after freeze-thaw

Formulation (100 mL): coenzyme Q₁₀ 1.0 g, soybean phospholipids 1.2 g, HS₁₅ 3.0 g, injectable oils 10 g (soybean oil/medium-chain oil = 15/85 (w/w)), EDTA-2Na 0.05 g, an appropriate amount of a cryoprotectant, and injectable water as the balance.

Preparation process: 1) heating the injectable oils, phospholipids and HS₁₅ in a preparation tank till 50°C, strongly stirring till dissolved, and adding the drug, stirring till dissolved (adding active carbon, if necessary; and removing the pyrogens by conventional methods); 2) adding the cryoprotectant and EDTA-2Na into injectable water in another preparation tank, stirring at 50°C for 5 min till completely dissolved (adding active carbon, if necessary; and removing the pyrogens by conventional methods); 3) adding the product coming from step 2) into the product coming from step 1), stirring at 50°C to obtain the primary emulsion; 4) passing the primary emulsion (liquid) through a microfluidizer, adjusting the homogenizing pressure to 5000 psi at first, and then adjusting to 14000 psi, repeating the homogenization to obtain homogeneous emulsion.

An appropriate amount of various cryoprotectants are added into the emulsions, respectively, and stirred for dissolving homogeneously. The injectable coenzyme Q₁₀ emulsion was obtained after filtration with membrane, package, filling with nitrogen, sealing and sterilization, and then subjected to freeze-thaw experiments. The results are shown in Table 5.

**Table 5: Effects of different cryoprotectants on freeze-thaw stability of coenzyme Q₁₀ emulsions**

| Cryoprotectants | Average particle size of original emulsion (nm) | Average particle size of emulsion after freeze-thaw (nm) | Percentage of increase in particle size |
|---|---|---|---|
| No Cryoprotectant | 60.8 | 255.9 | 320% |
| Propanediol 5% | 61.5 | 74.7 | 21% |
| Propanediol 10% | 62.6 | 79.9 | 27.6% |
| PEG400 2.4% | 63.3 | 265.1 | 319% |
| Glycerol 2.4% | 57.5 | 236.5 | 311% |
| Trehalose 5% | 56.7 | 321.4 | 467% |
| Trehalose 10% | 54.5 | 286.7 | 426% |
| Lactose 10% | 57.6 | 325 | 464% |
| Maltose 5% | 58.8 | 157.8 | 168% |
| Maltose 10% | 54.5 | 156.1 | 186% |
| Mannitol 6% | 56.8 | 114.4 | 101% |
| glucose 1.5% | | | |
| Mannitol 6% | 55.9 | 139.6 | 150% |
| glucose 3% | | | |
| Mannitol 5% | 62.4 | 237.6 | 281% |
| sucrose 10% | | | |
| Mannitol 6% | 52.7 | 146 | 177% |
| maltose 1.5% | | | |
| Mannitol 2% | 63.2 | 263.9 | 318% |
| glucose 4% | | | |
| lactose 8% | | | |
| Mannitol 2% | 59.3 | 78.6 | 32.5% |
| glucose 4% | | | |
| maltose 8% | | | |

It can be seen from Table 5, propanediol is a preferred cryoprotectant of alcohols. Among the cryoprotectants of saccharides, the percentage of increases in particle size was less than 200% when maltose alone, the combination of mannitol 6% and glucose 1.5%, the combination of mannitol 6% and glucose 3%, the combination of mannitol 6% and maltose 1.5%, the combination of mannitol 2%, glucose 4% and maltose 8% were used. In particular, the percentage of increase in particle size was less than 50% when the combination of mannitol 2%, glucose 4% and maltose 8% was used.

### Example 5

Formulation (100 mL): coenzyme Q₁₀ 0.1 g, soybean phospholipids 0.6 g, HS₁₅ 1.8 g, injectable oils 5 g (soybean oil/medium-chain oil = 15/85 (w/w)), glucose 1.5 g, mannitol 6 g and injectable water as the balance.
(1) heating the injectable oils, soybean phospholipids and HS₁₅ in a preparation tank till 50°C, strongly stirring till dissolved, and adding coenzyme Q₁₀, stirring till dissolved;
(2) adding glucose and mannitol into injectable water in another preparation tank, stirring at 50°C for 5 min till completely dissolved;
(3) adding the product coming from step 2) into the product coming from step 1), stirring at 50°C to obtain the primary emulsion;
(4) passing the primary emulsion (liquid) through a microfluidizer, adjusting the homogenizing pressure to 5000 psi at first, and then adjusting to 14000 psi, repeating the homogenization to obtain homogeneous emulsion, and then filtering with membranes, packaging, filling nitrogen, sealing and sterilizing to obtain coenzyme Q₁₀ emulsion for injection. Upon determination, the average particle size of the emulsion was 46.8 nm, and was 49.5 nm after freeze-thaw. In other words, the emulsions with small particle size and greatly increased freeze-thaw resistance can be obtained by reducing the proportion of the oil phase.

An emulsion was then prepared by replacing HS₁₅ with TPGS, which had an average particle size of 36 nm and an average particle size of 37 nm after freeze-thaw.

### Example 6

Formulation (100 mL): coenzyme Q₁₀ 0.25 g, soybean phospholipids 0.3 g, HS₁₅ 1.8 g, injectable oils 5 g (soybean oil/medium-chain oil = 15/85 (w/w)), glucose 1.5 g, mannitol 6 g and injectable water as the balance.

The process was carried out as described in example 5, excepting the temperature in step (2) was 30 °C. The emulsion obtained had an average particle size of 51.0 nm and 53.3 nm before and after freeze-thaw, respectively.

The emulsion prepared by replacing HS₁₅ with TPGS had an average article size of 43 nm and an average particle size after freeze-thaw of 49 nm.

### Example 7

Formulation (100 mL): coenzyme Q₁₀ 0.25 g, soybean phospholipids 0.3 g, HS₁₅ 1.8 g, injectable oils 5 g (soybean oil/medium-chain oil = 15/85 (w/w)), glycerol 2.4% and injectable water as the balance.

The process was carried out as described in example 5, excepting glycerol was added into injectable water in step (2). The average particle size of the emulsion thus obtained was 63.1 nm. The average particle size after freeze-thaw was 62.8 nm.

The emulsions prepared by replacing HS₁₅ with TPGS had an average article size of 38 nm and an average particle size after freeze-thaw of 40 nm.

### Example 8

Formulation (100 mL): coenzyme Q₁₀ 0.5 g, soybean phospholipids 1.2 g, HS₁₅ 3.0 g, injectable oils 10 g (soybean oil/medium-chain oil = 15/85 (w/w)), propanediol 5.0 g, EDTA-2Na 0.05g and injectable water as the balance.

The process was carried out as described in example 5, excepting propanediol and EDTA-2Na were added into injectable water in step (2). The average particle size of the emulsion thus obtained was 58.9 nm. The average particle size after freeze-thaw was 61.5 nm.

The emulsions prepared by replacing HS₁₅ with TPGS had an average particle size of 53 nm and an average particle size after freeze-thaw of 57 nm.

### Example 9

Formulation (100 mL): coenzyme Q₁₀ 1.0 g, soybean phospholipids 1.2 g, HS₁₅ 3.0 g, injectable oils 10 g (soybean oil/medium-chain oil = 15/85 (w/w)), propanediol 5.0 g, EDTA-2Na 0.05g and injectable water as the balance.

The process was carried out as described in example 5, excepting propanediol and EDTA-2Na was added into injectable water in step (2). The average article size of the emulsion thus obtained was 66.9 nm. The average particle size after freeze-thaw was 64.2 nm.

The emulsions prepared by replacing HS₁₅ with TPGS had an average particle size of 72 nm and an average particle size after freeze-thaw of 76 nm.

### Example 10

Formulation (100 mL): coenzyme Q₁₀ 1.0 g, lecithin 1.0 g, HS₁₅ 2.0 g, poloxamer188 1.0 g, injectable oils 10 g (soybean oil/medium-chain oil = 15/85 (w/w)), propanediol 5.0 g, EDTA-2Na 0.05g and injectable water as the balance.
(1) heating the injectable oils, lecithin and HS₁₅ in a preparation tank till 50°C, strongly stirring till dissolved, and adding coenzyme Q₁₀, stirring till dissolved;
(2) adding poloxamer188, propanediol and EDTA-2Na into injectable water in another preparation tank, stirring at 50°C for 5 min fill completely dissolved;
(3) adding the product coming from step 2) into the product coming from step 1), stirring at 50°C to obtain the primary emulsion;
(4) passing the primary emulsion (liquid) through a microfluidizer, adjusting the homogenizing pressure to 5000 psi at first, and then adjusting to 14000 psi, repeating the homogenization to obtain homogeneous emulsion, and then filtering with membranes (for sterilization), packaging, filling with nitrogen, sealing and sterilizing (via moist heat sterillization or filtration sterilization) to obtain coenzyme Q₁₀ emulsion for injection. Upon determination, the average particle size of the emulsion was 62.6 nm. The average particle size after freeze-thaw was 63.3 nm.

The process was carried out as described above, excepting the amount of injectable oils increased to 15g. The emulsion thus obtained had an average particle size of 68 nm, and an average particle size after freeze-thaw of 71 nm.

The process was carried out as described above, excepting the amount of injectable oils increased to 20g. The emulsion thus obtained had an average particle size of 73 nm, and an average particle size after freeze-thaw of 78 nm.

### Example 11

A homogeneous emulsion was prepared with the same formulation and the process as described in example 10, excepting the latter homogenization pressure in step (4) was adjusted to 10000 psi instead of 14000 psi. Upon determination, the average particle size of the emulsion thus obtained was 91.6 nm. The average particle size after freeze-thaw was 93.9 nm.

### Example 12

A homogeneous emulsion was prepared with the same formulation and the process as described in example 11, excepting the homogenization pressure in step (4) was adjusted to 5000 psi. Upon determination, the average particle size of the emulsion thus obtained was 128.2 nm. The average particle size after freeze-thaw was 132.9 nm.

### Example 13: Freeze-thaw experiments on coenzyme Q₁₀ emulsion

The freeze-thaw experiments on coenzyme Q₁₀ emulsion were performed according to "Technical Guidelines in Chemical Drugs Stability" issued by SFDA, and comprised the steps of: freezing at (-20)-(-10)ºC for 2 days, then observing under the accelerated conditions at 40°C for 2 days. This freeze-thaw cycle was repeated for 3 times. The results are listed in Table 6.

**Table 6: Results of freeze-thaw experiments on coenzyme Q₁₀ emulsion**

| Cycle number | Example 5: Average particle size (nm) | Example 6: Average particle size (nm) | Example 7: Average particle size (nm) | Example 10: Average particle size (nm) |
|---|---|---|---|---|
| 0 | 46.8 | 51.0 | 63.1 | 62.6 |
| 1 | 48.9 | 51.9 | 63.8 | 61.7 |
| 2 | 50.1 | 54.5 | 59.3 | 63.3 |
| 3 | 52.1 | 55.6 | 64.9 | 65.7 |

It can be seen from Table 6, based on a large number of studies and experiments, the coenzyme Q₁₀ emulsions prepared with relative reasonable process had good freeze-thaw stability, no matter the composite emulsifier consists of two emulsifiers or three emulsifiers.

### Example 14: Dilution experiments on coenzyme Q₁₀ emulsion

The diluents for diluting emulsions for intravenous injection mainly include physiological saline and 5% glucose injection in clinic. The following experiments were performed for detecting the physical stability of the coenzyme Q₁₀ emulsions after dilution using these diluents.

Method: precisely taking 2 aliquots (2mL each) of coenzyme Q₁₀ emulsion coming from example 10, placing each in 10 mL volumetric flask, and adding respectively physiological saline and 5% glucose injection till predetermined volume, mixing, transferring into ground tubes with stoppers; meanwhile, precisely taking 2 aliquots (1 mL each) of coenzyme Q₁₀ emulsion, placing each in 10 mL volumetric flask, and subjecting to the same process as described above; and placing the four aliquots in a water bath at a constant temperature of 30°C, sampling after 0, 2, 4, 6, 8, 10 and 12 hours; detecting the average particle sizes and distribution, and determining the particle size changes of the emulsions during the period after mixed with the diluents. The results are listed in Table 7.

**Table 7: Results of dilution experiments on coenzyme Q₁₀ emulsion**

| Time (h) | Average particle size (nm) Diluting with 5% glucose injection | Average particle size (nm) Diluting with 0.9% NaCl injection |
|---|---|---|
| 0 | 73.2 | 77.4 |
| 2 | 65.5 | 71.4 |
| 4 | 65.8 | 75.1 |
| 6 | 67.2 | 69.1 |
| 8 | 63.1 | 66.9 |
| 10 | 68.8 | 66.4 |
| 12 | 70.4 | 68.5 |
| Average particle size (nm) | 67.7 | 70.7 |
| RSD(%) | 4.99% | 5.92% |

It can be seen from the results, the average particle size was substantially constant during the period of 12h after mixed with glucose injection and physiological saline, respectively, exhibiting good compatible stability

### Example 15: Photostability test on different preparations

The ethanol solutions of coenzyme Q₁₀ with concentrations of 0.1 mg/mL, 0.5 mg/mL and mg/mL, and nano-emulsions of coenzyme Q₁₀ with concentrations of 0.1 mg/mL, 0.5 mg/mL, 1 mg/mL and 10 mg/mL were prepared, packaged in vials and sealed. The vials were then placed in an artificial climate box at a temperature of 25°C. The forced photolysis experiments were carried out at 2000 Lux, 3000 Lux, 4000 Lux, 5000 Lux and 6000 Lux. The results are listed in Tables 8-12.

**Table 8: Photolysis kinetics parameters of coenzyme Q₁₀ preparations under 2000 Lux**

| Concentration (mg/mL) | Preparations | regression equation | r | *t*_{½} (h) | *t*_{½} ratio emulsion/solution |
|---|---|---|---|---|---|
| 0.1 | Solution | LnC = 4.6417 - 0.0259 t | 0.9963 | 26.8 | |
| 0.1 | Emulsion | LnC = 3.7822 - 0.0242 t | 0.9983 | 28.6 | 1.07 |
| 0.5 | Solution | Ln*C* = 4.6439 - 0.0240 t | 0.9967 | 28.9 | |
| 0.5 | Emulsion | Ln*C* = 4.4824 - 0.0151 t | 0.9915 | 45.9 | 1.59 |
| 1 | Solution | Ln*C* = 4.6322 - 0.0234 t | 0.9940 | 29.6 | |
| 1 | Emulsion | Ln*C* = 4.4781- 0.0073 t | 0.9974 | 94.9 | 3.21 |
| 10 | Example 10 | Ln*C* = 4.4781 - 0.0023 t | 0.9924 | 301.3 | |

**Table 9: Photolysis kinetics parameters of coenzyme Q₁₀ formulations under 3000 Lux**

| Concentration (mg/mL) | Preparations | regression equation | r | *t*_{½} (h) | *t*_{½} ratio emulsion/solution |
|---|---|---|---|---|---|
| 0.1 | Solution | LnC = 4.6301- 0.0388 t | 0.9966 | 17.9 | |
| 0.1 | Emulsion | LnC = 3.8022 - 0.0382 t | 0.9991 | 18.1 | 0.94 |
| 0.5 | Solution | LnC = 4.6631 - 0.0339 t | 0.9972 | 20.4 | |
| 0.5 | Emulsion | LnC = 4.5489 - 0.0306 t | 0.9944 | 22.6 | 1.11 |
| 1 | Solution | LnC = 4.6528 - 0.0278 t | 0.9946 | 24.9 | |
| 1 | Emulsion | LnC = 4.4885 - 0.0095 t | 0.9983 | 72.9 | 2.93 |
| 10 | Example 10 | LnC = 4.4885 - 0.0036 t | 0.9953 | 192.5 | |

**Table 10: Photolysis kinetics parameters of coenzyme Q₁₀ formulations under 4000 Lux**

| Concentration (mg/mL) | Preparations | regression equation | r | *t*_{½} (h) | *t*_{½} ratio emulsion/solution |
|---|---|---|---|---|---|
| 0.1 | Solution | LnC = 4.6176 - 0.0493 t | 0.9967 | 14.1 | |
| 0.1 | Emulsion | LnC = 3.8297 - 0.0502 t | 0.9933 | 13.8 | 0.98 |
| 0.5 | Solution | Ln*C* = 4.6373 - 0.0443 t | 0.9964 | 15.6 | |
| 0.5 | Emulsion | LnC = 4.5196 - 0.0423 t | 0.9964 | 16.8 | 1.08 |
| 1 | Solution | LnC = 4.6377 - 0.0357 t | 0.9938 | 19.4 | |
| 1 | Emulsion | Ln*C* = 4.4557 - 0.0125 t Ln*C* = | 0.9963 | 55.4 | 2.86 |
| 10 | Example 10 | 4.5099 - 0.0052 t | 0.9970 | 133.3 | |

**Table 11: Photolysis kinetics parameters of coenzyme Q₁₀ formulations under 5000 Lux**

| Concentration (mg/mL) | Preparations | regression equation | r | *t*_{½} (h) | *t*_{½} ratio emulsion/solution |
|---|---|---|---|---|---|
| 0.1 | Solution | LnC = 4.6488 - 0.0599 t | 0.9924 | 11.6 | |
| 0.1 | Emulsion | LnC = 3.7547 - 0.0634 t | 0.9988 | 10.9 | 0.94 |
| 0.5 | Solution | Ln*C* = 4.6715 - 0.0535 t | 0.9947 | 13.0 | |
| 0.5 | Emulsion | Ln*C* = 4.5365 - 0.0505 t | 0.9978 | 13.7 | 1.05 |
| 1 | Solution | Ln*C* = 4.6505 - 0.0495 t | 0.9986 | 14.0 | |
| 1 | Emulsion | Ln*C* = 4.4801 - 0.0148 t | 0.9959 | 46.8 | 3.34 |
| 10 | Example 10 | In*C*= 4.5112 - 0.0071 t | 0.9987 | 97.6 | |

**Table 12: Photolysis kinetics parameters of coenzyme Q₁₀ formulations under 6000 Lux**

| Concentration (mg/mL) | Preparations | regression equation | r | *t*_{½} (h) | *t*_{½} ratio emulsion/solution |
|---|---|---|---|---|---|
| 0.1 | Solution | LnC = 4.6242 - 0.0771 t | 0.9949 | 9.0 | |
| 0.1 | Emulsion | LnC = 3.8006 - 0.0811 t | 0.9982 | 8.5 | 0.94 |
| 0.5 | Solution | Ln*C* = 4.6359 - 0.0667 t | 0.9975 | 10.4 | |
| 0.5 | Emulsion | Ln*C* = 4.5590 - 0.0635 t | 0.9967 | 10.9 | 1.05 |
| 1 | Solution | Ln*C* = 4.6345 - 0.0596 t | 0.9965 | 11.6 | |
| 1 | Emulsion | Ln*C* = 4.4803 - 0.0167 t | 0.9960 | 41.5 | 3.58 |
| 10 | Example 10 | Ln*C* = 4.5162 - 0.0091 t | 0.9953 | 76.2 | |

It can be seen from tables 8-12, the photolysis of coenzyme Q₁₀ was concentration-dependent. The photolysis rate decreased along with the increase of the original concentration. Coenzyme Q₁₀ emulsions have increased photostability drugalong with the increase of the concentrations, as compared with the corresponding coenzyme Q₁₀ solutions. This result had never been reported before. The suitable concentration of coenzyme Q₁₀ in emulsion was ≥ 1 mg/mL.

### Example 16: Protective effects of coenzyme Q₁₀ emulsion on cardiotoxicity of adriamycin.

Coenzyme Q₁₀ can reduce the cardiotoxicity induced by adriamycin. In this experiment, adriamycin-induced myocardial injury model in rat was constructed for detecting the effects of coenzyme Q₁₀ nanoemulsion of protecting rats from adriamycin-induced myocardial injury.

40 Wistar rats (250±15g, F/M = 1:1) were randomly divided into five groups (8 animals each group): negative control group, ADM group, ADM + coenzyme Q₁₀ (low dosage) group, ADM + coenzyme Q₁₀ (medium dosage) group and ADM + coenzyme Q₁₀ (high dosage) group. The control group received intraperitoneal injection of physiological saline at 10mL/kg/d for 9 days. The ADM group received intraperitoneal injection of ADM at 3 mg/kg every other day for 5 times in total (9 days). The ADM + coenzyme Q₁₀ (low, medium and high dosages) groups respectively received tail vein injection of coenzyme Q₁₀ t 1.5, 3.0 and 6.0 mg/kg/d for 9 days, and ADM was administered at 3 mg/kg every other day for 5 times in total (9 days) (10 min after coenzyme Q₁₀ injection, intraperitoneal injection of ADM 3mg/kg was performed). The rats were sacrificed on the 10^{th} day for histopathological examination and detection for the contents of malonaldehyde and SOD. The histopathological examination was performed by the steps of: taking myocardial tissue from the left ventricular, fixing with 10% formaldehyde, slicing according to the conventional methods, and staining with HE. The injuries were classified into four grades according to the criteria of Rona *et*. *al*. The results are listed in Tables 13 and 14.

**Table 13 Pathological results indicating the protective effects of coenzyme Q₁₀ emulsion on myocardial injury induced by adriamycin (n =8)**

| Groups | Pathological classification of myocardial injury | | | | |
|---|---|---|---|---|---|
| | 0 | I | II | III | IV |
| Control | 8 | - | - | - | - |
| ADM group | - | - | 3 | 5 | - |
| ADM + coenzyme Q₁₀ (low dosage) | - | 3 | 5 | - | - |
| ADM + coenzyme Q₁₀ (medium dosage) | - | 4 | 4 | - | - |
| ADM + coenzyme Q₁₀ (high dosage) | - | 5 | 3 | - | - |

It can be seen from Table 13, tail vein injection of coenzyme Q₁₀ emulsions could alleviate adriamycin-induced myocardial injury.

**Table 14 Changes of MDA and SOD contents in myocardium (n =8)**

| Groups | myocardial MDA (nmol/mg prot) | myocardial SOD (U/mg prot) |
|---|---|---|
| Control | 4.76 ± 0.65 | 426.26 ± 11.29 |
| ADM group | 8.99 ± 1.17^{a} | 330.02 ± 15.39^{a} |
| ADM + coenzyme Q₁₀ low dosage) | 5.20 ± 1.07^{b} | 378.93 ± 6.50 |
| ADM + coenzyme Q₁₀ (medium dosage) | 5.09 ± 1.03^{b} | 411.56 ± 13.28^{b} |
| ADM + coenzyme Q₁₀ (high dosage) | 4.91 ± 0.78^{b} | 415.00 ± 6.50^{b} |

| | | |
|---|---|---|
| Note: a: P<0.05 compared with control group; b: P<0.05, compared with ADM group | | |

It can be seen from Table 14, upon intraperitoneal injection of adriamycin, the myocardial MDA content in rats significantly increased as compared with the controls (P<0.05), while the myocardial SOD content significantly decreased as compared with the controls (P<0.05). After tail vein injection of coenzyme Q₁₀ emulsions at different dosages, the myocardial MDA content in rats increased slightly without significant difference as compared with the controls, and the difference between each dosage group and ADM group was significant (P<0.05). The myocardial SOD content in rats decreased slightly without significant difference as compared with the controls, and the difference between medium/high dosage groups and ADM group was significant (P<0.05). Coenzyme Q₁₀ emulsions could effectively reduce the cardiotoxicity of adriamycin, protect SOD activity in the rats with injured myocardium, and thus enhance the function of endogenous reactive oxygen species scavenging system and reduce the generation of MDA.

The alterations of rat electrocardiograms are shown in Figures 1-3. Compared with electrocardiogram of Figure 1 under normal conditions, electrocardiogram 2 of Figure 2 for ADM group showed absent P-wave, flattened T-wave, obviously reduced ST-T segment, and decreased QRS waves. Electrocardiogram 3 of Figure 3 for coenzyme Q₁₀ groups showed markedly restored P-wave, T-wave, ST-T segment and QRS waves.

Myocardial tissue slice (stained with HE) indicated: there were no changes in the myocardial tissues of controls; myocardial tissues from rats received intraperitoneal injection of ADM were seriously injured, representing as focal necrosis of cardiac myocytes at endocardium, disappearance of cross striation, disappearance of cell nucleus, irregular arrangement of myocardial fibers in each ventricular walls, and large area of focal necrosis. Tail vein injection of coenzyme Q₁₀ nana-emulsions alleviated adriamycin-induced myocardial injury.

### Example 17: Primary evaluation on targeting ability for coenzyme Q₁₀ emulsions

Administration and sampling: mice fasted 12h were randomly divided into four groups and respectively received tail vein injection of coenzyme Q₁₀ solution and nano-emulsions with different particle sizes. Each group had 3 mice at each time point The administration dosage was 4.0 mg-kg⁻¹. Blood samples were taken from orbits at different time points of 10 min, 30 min, 1 h, 2 h, 6 h and 12 h after administration. The plasma samples were separated by centrifugation. Heart, liver and brain were taken rapidly, washed with cold physiological saline, dried with filter paper and cryopreservated at -20°C.

Coenzyme Q₁₀ contents in the tissues were quantitatively detected via HPLC.

The instruments used were HPLC device (Dalian Elite Analytical Instruments Co., Ltd.), UV200011 UV-VIS Changeable Wavelength Detector (Dalian Elite Analytical Instruments Co., Ltd.), and HW2000 chromatography data processing workstation (Dalian Elite Analytical Instruments Co., Ltd.).

Chromatographic column: Hypersil BDS C18 (200 mm×4.6 mm, 5 µm, Dalian Elite Analytical Instruments Co., Ltd.); mobile phase: methanol-anhydrous ethanol (20:80, v/v); column temperature 30°C; flowing rate 1.0 mL/min; detection wavelength 275 nm; loading volume: 20 µL.

Pharmacokinetics program 3p87 was used for processing data so as to obtain AUC of each tissue. The results are listed in Table 15.

**Table 15 AUC of each tissue and targeting efficiency of coenzyme Q₁₀**

| Tissue | Heart | | Liver | | Brain | |
|---|---|---|---|---|---|---|
| (µg-h-g⁻¹) | AUC | Relative adsorption rate | AUC | Relative adsorption rate | AUC | Relative adsorption rate |
| Solution | 6.602 | - | 74.809 | - | 54.354 | - |
| Example 10 | 28.031 | 4.246 | 100.098 | 1.338 | 65.051 | 1.197 |
| Example 11 | 25.328 | 3.836 | 89.785 | 1.201 | 59.389 | 1.093 |
| Example 12 | 27.340 | 4.141 | 104.636 | 1.399 | 55.689 | 1.025 |

It can be seen from Table 15, when the particle size of the emulsion was less than 150nm, the relative adsorption rates (re) in heart, liver and brain were all larger than 1, although the particle sizes of the emulsions were significantly different (62.6 nm, 91.6 nm and 128.2 nm in examples 10, 11 and 12), indicating the targeting ability of the emulsions to those tissues. The targeting efficiencies were found as heart >> liver > brain without significant differences. This result provided a basis for industrial production in future as well as control for alteration of emulsions during storage. This result had never been reported before.

### Example 18: Combination of three composite emulsifiers for preparing emulsions

Cucurbitacin (commercially available raw cucurbitacin, the total cucurbitacin contents in the emulsion was 0.1mg/ml) was used as a model drug for screening the formulations. The oil phase was 10% MCT (w/v). The preparation process was used as descried in example 10. The results were compared with that of the emulsion using composite emulsifier of two emulsifiers. The particle sizes detected were Gaussian volume diameter.

**Table 16: Experiment results of cucurbitacin emulsions prepared with different composite emulsifiers**

| Formulation | 1 | 2 | 3 | 4 | 5 | 6 |
|---|---|---|---|---|---|---|
| Components (%w/v) | SPC 1 | SPC 1 | SPC 1 | SPC0.2 TPGS 3.5 VE 1 F68 0.2 | SPC 2 TPGS 1 VE 1 F68 0.2 | SPC 1 TPGS 0.3 VE 1 F68 2 |
| | HS₁₅ 2 | HS₁₅ 2 | HS₁₅ 3 | | | |
| | VE 1 | VE 1 | VE 1 | | | |
| | F68 0 | F68 1 | F68 1 | | | |
| Particle size before sterilization (nm) | □ 74.7 | □ 54.3 | □ 45.8 | 48.6 46.2 | 68.3 66.5 | 67.7 72.6 |
| | □ 74.8 | □ 52.5 | □ 46.3 | | | |
| | □ 64.5 | □ 57.9 | | | | |
| Particle size after sterilization (sterilization for 30 min) | □ 77.1 | □ 54.0 | □ 80.3 | 51.6 55.3 | 76.6 78.2 | 70.3 75.6 |
| | □ 72.2 | □ 54.9 | □ 81.6 | | | |
| | □ 63.6 | □ 62.0 | | | | |
| Particle size after freeze-thaw (nm) | Undetectable | 53.2 | 76.8 | 56.2 52.7 | 71.8 78.3 | 72.8 80.1 |
| | | | | | | |
| | | 55.1 | 75.1 | | | |
| | | 58.6 | | | | |

| | | | | | | |
|---|---|---|---|---|---|---|
| Note: Vitamin E (VE) in the formulation was used as an anti-oxidant. Cucurbitacin in the emulsions without VE was unstable. The article size of the emulsions reduced 6% after sterilization. The emulsions with the addition of VE did not exhibit any changes. ①, ② and ③ represent the numbers for repeating the experiment | | | | | | |

When TPGS was removed from formulation 6, the emulsion thus prepared lost freeze-thaw resistance. When TPGS was replaced with DSPE-PEG2000 with other unchanged components, the particle sizes of the emulsions before and after sterilization were 55.3 nm and 60.6 nm, respectively, and the particle size of the emulsions was 62.3 nm after freeze-thaw, which exhibited that DSPE-PEG2000 also had good effect of resisting freeze-thaw.

Based on formulation 2 in Table 6, the thermo-resistance and freeze-thaw resistance of the emulsions prepared by different devices, including NANOMIZER (Japan), IMCROFLUIDIZER (high pressure micro-fluidiang nano-distributor, made in US), Nano-microemutsifying devices (China, DSS Serious), EKATONANOMIX (Germany), and Niro Soavi NS1001 L (high pressure homogenizer, made in Italy) were detected. The results did not show significant difference, demonstrating the present invention can be applied to different devices in different dispersion principles (in the case of the classical formulation, i.e., using lecithin as the emulsifier, the devices used may influence the particle size and distribution of the emulsions, and the emulsions could not meet the requirement of thermo-resistance and freeze-thaw resistance).

Furthermore, the pH value range of 3-9 was also detected. The results showed the value of pH did not have significant effects on the particle size of the emulsions. This advantage is not possessed by the prior classical emulsions. In view of this, the present invention is especially suitable for preparing emulsions of pH-sensitive drugs. When the oil phase was replaced with mixed oils at different ratios, such as LCT/MCT 10:90,20:80,30:70,40:60,50:50,60:40, 70:30, 80:20, 90:10, 100:0, the emulsions thus obtained could also resist heat and freeze-thaw. In the experiments on oil phase concentrations, it was found when the oil phase concentration exceeded 20%, the emulsion obtained could not resist heat and freeze-thaw. Thus, the oil phase concentration of less than or equal to 20% is preferred.

When the content of F68 (poloxamer) in formulation 6 was altered to 5% or 10% (w/v) and other components remained the same, the average particle sizes of the emulsions were 63 nm and 38 nm, respectively No obvious change of particle size was observed after freeze-thaw

### Example 19:

The similar experimental results were obtained without obvious change observed, by replacing SPC in example 18 with EPC with no change to other components.

### Example 20:

The similar experimental results were obtained without obvious change observed, by replacing cucurbitacin in example 18 with cucurbitacin B (or dihydrocucurbitacin, isocucurbitacin B, cucurbitacin D, cucurbitacin E, cucurbitacin I, cucurbitacin Q) (HPLC area normalized purity> 90%) with no change to other components.

### Example 21:

The process of example 18 was taken with simple modification, wherein cucurbitacin is replaced by coenzyme Q₁₀ (the concentration of coenzyme Q₁₀ in the emulsion was 1%, i.e., the concentration of coenzyme Q₁₀ in the formulation was 10 mg/ml). The results are listed in Table 17.

**Table 17: Experimental results of coenzyme Q₁₀ emulsions prepared with different composite emulsifiers**

| Formulation | 1 | 2 | 3 | 4 | 5 | 6 |
|---|---|---|---|---|---|---|
| Components (% w/v) | SPC 1 | SPC 1 | SPC 1 | SPC 0.2 | SPC 2 | SPC 1 |
| | HS₁₅ 2 | HS₁₅ 1.5 | HS₁₅ 3 | TPGS 3.5 | TPGS1 | TPGS 0.3 |
| | F68 0 | F68 1 | F68 1 | F68 0.2 | F68 0.2 | F68 2 |
| Particle size before sterilization (nm) | 89.2 | 72.1 | 68.3 | 67.2 | 80.2 | 78.5 |
| Particle size after sterilization (nm) | - | 73.5 | 89.2 | 73.1 | 89.6 | 82.1 |
| Particle size after freeze-thaw (nm) | Undetectable | 78.1 | 92.5 | 72.6 | 87.2 | 91.3 |

The emulsion prepared with the composite emulsifier of 1% SPC and 1% F68 but without HS₁₅ was also unendurable to freeze-thaw. In the case that SPC was not used and the composite emulsifier was consisted of HS₁₅ and F68 or TPGS and F68, the emulsions prepared were still poor in thermo-resistance and freeze-thaw resistance.

### Example 22:

The process was carried out as described in example 9, excepting the concentration of coenzyme Q₁₀ was altered to 0.1% and 0.5%, and SPC was replaced by EPC. By the detection method as described in example 19, the same result with no significant changes was obtained.

### Example 23: Vitamin K₁ emulsion (10mg/ml)

The emulsion exhibited particle size of less than 100 nm and could resist to heat and freeze-thaw, when coenzyme Q₁₀ used in example 21 was replaced with vitamin K₁ at a concentration of 1% (concentration of the preparation = 10mg/ml) and other components remained the same.

### Example 24: Vitamin K₁ emulsion (1mg/ml, 5mg/ml)

The same result was obtained when vitamin K₁ concentration in example 23 was altered to 0.1% (concentration of the preparation = 1mg/ml) or 0.5% (concentration of the preparation = 5mg/ml) and SPC was replaced with EPC.

### Example 25: Diisopropylphenol emulsion

The emulsifier components of formulations 2 and 4 in Table 16 of example 18, and oil phase of 10% mixed oil (LCT/MCT, 15/85, the oil phase of example 9) were used for preparing the diisopropylphenol emulsion (concentration of 1%) of the present invention. The particle sizes of those emulsions were 76 nm and 68 nm, respectively. Another diisopropylphenol emulsion was prepared according to the commercial formulation (diisopropylphenol 1%, LCT 1%, SPC 1.2% and glycerol 2.2%). The particle size of the emulsion prepared was 187 nm.

The above three preparations were placed at 40°C for 10 days. The preparations with the formulation of the present invention were milk-white in appearance, while the emulsion with the commercial formulation and the commercial product turned to light yellow in color. The results of freeze-thaw experiments showed, the commercial diisopropylphenol emulsion (AstraZeneca) could not resist to freeze-thaw, while the appearance and particle size of the emulsions of the present invention were substantially unchanged after freeze- thaw, which demonstrated having a better stability than the commercial products.

### Example 26: Emulsion of Brucea javanica oil

The emulsifier components of formulations 2 and 4 in Table 16 of example 18, and oil phase of 10% *Brucea javanica* oil were used for preparing the *Brucea javanica* oil emulsion of the present inventions. The particle sizes of those emulsions were 81 nm and 73 nm, respectively. Meanwhile the particle size of commercial *Brucea javanica* oil emulsion (Shenyang Yaoda Pharmaceutical Co., Ltd.) was determined as 266 nm. The above three preparations were subjected to freeze-thaw. The results indicated the particle sizes of the present emulsions were 83 nm and 71 nm, respectively, while the particle size of the commercial *Brucea javanica* oil emulsion was undetectable, due to the serious damage to the emulsion system. The results indicated the preparation of the present invention was much better than the commercial product.

### Example 27: Alprostadil Emulsion

The emulsifier components of formulations 2 and 4 in Table 16 of example 18, and oil phase of 10% mixed oil (LCT/MCT, 15/85, the oil phase of example 9) were used for preparing the alprostadil emulsion of the present invention. After filtration sterilization, the particle sizes of those emulsions were 66 nm and 62 nm, respectively. Meanwhile the particle size of commercial alprostadil emulsion (Trade name: Kaishi, Beijing Tide Pharmaceutical Co., Ltd.) was determined as about 233 nm. The above three formulations were subjected to freeze-thaw. The results indicated the particle sizes of the present emulsions were 69 nm and 63 nm, respectively, while the particle size of the commercial alprostadil emulsion was undetectable due to the serious damage to the emulsion system. The results indicated the preparation of the present invention was much better than the commercial product. The stimulation caused by the present emulsion was also reduced.

### Example 28: Emulsion of Wufu Xinnaokang

Wufu Xinnaokang soft capsules (also known as Wufu Xinnaoqing, Xinnaoqing/Xinnaokang) were manufactured by Shineway Pharmaceutical Group Limited, and mainly comprised refined safflower oil, vitamin B₆, vitamin E, borneol, etc.

The emulsifier components of formulations 2 and 4 in Table 16 of example 18 were used for preparing Wufu Xinnaokang emulsions respectively containing 10%, 5% and 1% refined safflower oil. The particle sizes of those emulsions were 91 nm 73 nm and 56 nm, respectively. The above three preparations were subjected to freeze-thaw. The results showed there was no significant change of the particle size for the three Wufu Xinnaokang emulsions.

### Example 29: Butylphthalide emulsion

The emulsifier components of formulation 2 in Table 16 of example 18, oil phase of 10% mixed oil (LCT/MCT, 15/85, w/w), and 1% butylphthalide were used for preparing the butylphthalide emulsion of the present invention. The particle size of the obtained emulsion was about 70 nm. The particle size of the emulsion after freeze-thaw was 73 nm without significant changes with before freeze-thaw.

### Example 30: Elemenum emulsion

Elemene is a composition comprising β-, γ- and δ-elemenes from zedoary turmeric oil as the main components. The existing emulsion is manufactured from elemene, soybean phospholipid and cholesterol (Approval No. WS-258(X-218)-93(1)) with a gauge of 20ml:0.1g, i.e., 0.5%. Because the existing prescription has some defects and exhibits serious stimulation in clinic, Patent No. ZL02155072.7 provides an emulsion, which comprises (in 100mL) elemene 0.05-0.25%, injectable soybean oil 10-30%, injectable yolk lecithin 1.0-1.5% or injectable soybean lecithin 0.8-1.5%, injectable glycerol 2.0-2.5% and injectable water as the balance. An emulsion was prepared according the above patent and subjected to freeze-thaw experiments. The results showed the emulsion was unendurable in the freeze-thaw experiments.

In the present example, the emulsifier components of formulation 2 in Table 16 of example 18 were used. The components of the oil phase are listed in Table 18.

**Table 18 Components of the oil phase**

| Formulation No. | 1 | 2 | 3 |
|---|---|---|---|
| Elemene (%) | 0.05 | 0.5 | 1 |
| LCT(%) | 5 | 8 | 2 |
| MCT (%) | 5 | 2 | 8 |

The process of example 9 was used for preparing the emulsions. The particle sizes of the emulsion prepared according to formulations 1, 2 and 3 were 63 nm, 58 nm and 67 nm, respectively, and were all less than 70 nm without significant changes after freeze-thaw experiments. As shown in the mouse-twisting experiments, the present preparation caused much less stimulation than the commercial preparations. (Note: the mouse-twisting experiments is described as: experimental animal: Kunming mouse, 10 animals for each group, F/M = 1:1; the preparations were diluted to a drug concentration of drug 2mg/ml; dosage and administration route: 0.5 ml per animal, intraperitoneal injection; the twisting numbers of the mice were counted within 0.5h. The average twisting numbers of the mice administered commercial preparations or the preparation of the present invention were 10.2, and 2.6, respectively).

### Example 31: Emulsion of dexamethasone palmitate

The existing dexamethasone palmitate preparation is the emulsion with a trade name of Limethason, which contains 4.0 mg dexamethasone palmitate as the active component in a 1 ml ampoule. The inventor found the emulsion was also unendurable to freeze-thaw.

The emulsifier components of formulation 2 in Table 16 of example 18, and oil phase of 10% mixed oil (LCT/MCT, 15/85, w/w) were used for preparing the dexamethasone palmitate emulsion of the present invention (4.0 mg active component/1 mL), whose particle size was less than 100 nm (about 57 nm). The particle size of the emulsion after freeze-thaw was 61 nm without significant changes with before freeze-thaw.

### Example 32: Emulsions of lipid-soluble vitamins

lipid-soluble vitamins are essential nutrients for humans. Clinically critical patients often require supplement of lipid-soluble vitamins for survival. However, lipid-soluble vitamins are insoluble in water, and have relatively low bioavailability for oral administration, thus are more suitable for intravenous injection. There are two commercial products of lipid-soluble vitamins: injections I and II (according to Standard II Issued by the Ministry, Volume 5, pages 85-90) with the following formulations:
Formulation I: vitamin A 69 mg (230,000 units); vitamin D₂ 1.0 mg (40,000 units); vitamin E 0.64 g (70,000 units); vitamin K₁ 20 mg; injectable soybean oil 100 g; injectable lecithin 12 g; glycerol 22.0 g; an appropriate amount of injectable water, 1000 mL in total.
Formulation II: vitamin A 99 mg (330,000 units); vitamin D₂ 0.5 mg (20,000 units); vitamin E 0.91 g (1,000 units); vitamin K₁ 15 mg; injectable soybean oil 100 g; injectable lecithin 12 g; glycerol 22.0 g; an appropriate amount of injectable water, 1000 mL in total.

The emulsions with above formulations were manufactured by Sino-Swed Pharmaceutical Corp. Ltd., and were found to be unendurable to freeze-thaw in the freeze-thaw experiments.

When the emulsifier, "injectable lecithin", in the formulations was replaced by a composite emulsifier consisting of "SPC 1 g, HS₁₅ 2g and poloxamer F68 1 g" without additional VE, the emulsions thus prepared had average particle sizes less than 100 nm (66 nm and 62 nm, respectively). The particle sizes of the emulsions after freeze-thaw experiments were 70 nm and 68 nm without significant changes with before. When the emulsifier, "injectable soybean oil", in the formulations was replaced by mixed oil consisting of "LCT/MCT, 15/85, w/w" and the emulsifier was the emulsifier shown in formulation 2 in Table 16 of example 18 (without additional VE), the emulsions thus prepared had average particle sizes less than 100 nm (58 nm and 60 nm, respectively). The particle sizes of the emulsions after freeze-thaw experiments were 63 nm and 66 nm without significant changes with before.

### Example 33: Emulsion of irisquinone

Irisquinone includes pallason A and pallason B with the following structures:

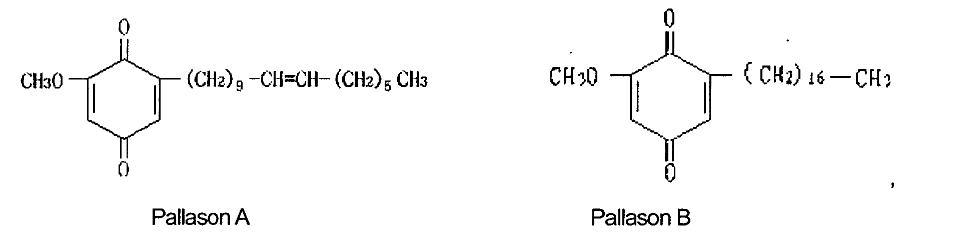

Which are similar to the following structure of coenzyme Q₁₀:

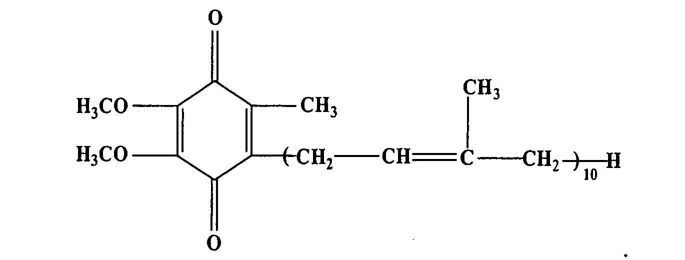

Irisquinone emulsion with thermo-resistance and freeze-thaw resistance was prepared according to example 9. The irisquinone concentration in the final preparation was 10 mg/mL. The average particle size of the emulsion thus prepared was 66 nm. The average particle sizes of the emulsion after sterilization and freeze-thaw were 68 nm and 69 nm, respectively When HS₁₅ in the formulation was replaced with TPGS, the average particle size was changed to about 53 nm, and the average particle sizes of the emulsion after sterilization and freeze-thaw were 51 nm and 56 nm, respectively.

0.2% DSPE-PEG₂₀₀₀ was added into the formulation. Therefore, the formulation was consisted of: irisquinone 1.0 g, lecithin 1.0 g, HS₁₅ 2.0g, poloxamer188 1.0 g, DSPE-PEG₂₀₀₀) 0.2 g, injectable oil 10 g (LCT/MCT, 15/85, w/w), glycerol 2.4 g, EDTA-2Na 0.05 g and injectable water as the balance (100 mL in total). The average particle size of the emulsion thus prepared was 56 nm. The average particle sizes of the emulsion after sterilization and freeze-thaw were 52 nm and 56 nm, respectively. Both emulsions prepared according to above two formulations had good thermo-resistance and freeze-thaw resistance. Similarly, the emulsion of each individual compound can be prepared according to the above formulation and process.

### Example 34:

The emulsions of fish oil (deep see fish oil, rich in polyunsaturated fatty acids and esters thereof, such as DHA), coix seed oil, zedoary turmeric oil, Bupleurum oil, Seabuckthom Seed oil, garlic oil, allicin, *Chuanxiong* rhizome oil, angelica oil, capsicum oil, ginger oil, celery seed oil, *Houttuynia cordata* oil, Evening primrose oil, perilla seed oil, batyl alcohol, tanshinone IIA, ligustilide, entecavir, anethol trithione, malotilate, homoharringtonine, demethylcantharidate, curcumine, cyclandelate, β-elemene, calcitriol, statins as hypolipidemic drugs (such as lovastatin and simvastatin, etc.), tocopherol nicotinate, gossypol, oridonin, fenofibrate, itraconazole, candesartan, hydroxycamptothecin, camptothecin derivatives (paclitaxel and the derivatives thereof, docetaxel and the derivatives thereof), finasteride, etoposide, ginsenoside, 20(S)-protopanaxadiol, ginsenoside Re, ginsenoside Rb1, ginsenoside Rg₂, 20(R)-ginsenoside Rg₃ and 20(S)-ginsenoside Rg₃ were prepared according to the process of example 18. The average particle sizes of these emulsions thus prepared were all less than 150 nm. The particle sizes of these emulsions after freeze-thaw experiments exhibited no significant difference from before freeze-thaw.

Based on the practice, the ratios of the lipophilic compounds or drugs to the C₆-C₂₈ oils are in the range of 1:0-1:10,000 w/w. When oily drug is used, no additional oil is required. Thus, the ratio of the drug to the C₆-C₂₈ oils is 1:0 w/w. When calcitriol is used, the concentration of calcitriol in the preparation is 1 µg/mL, the amount of the C₆-C₂₈ oils in the preparation is 1% (w/v), and thus the ratio of the drug to oils is 1:10,000 w/w. When 0.1% (w/v) oils were used, the ratio of the drug to oils is 1:1000 w/w.

When the phospholipids used in all above examples were replaced by other natural, semi-synthesized, or synthesized phospholipids, such as cardiolipin, phosphatidylinositol, phosphatidylglycerol, sphingomyelin (SM), phosphatidylserine (PS), hydrogenated lecithin, dipalmitoyl phosphatidyl choline (DMPC), dioleoyl phosphatidyl choline (DOPC), dilauroyl phosphatidylcholine (DLPC), phosphatidylethanolamine (PE), the same results were obtained for the emulsions prepared.

### Example 35:

The addition of co-emulsifiers of oleic acid, linoleic oil, linolenic acid, stearic acid, docosahexaenoic acid, cholic acid, deoxycholic acid, tocopherol, lipoic acid, sodium pyrosulfite, sodium sulfite, nitrogen, citric add and like, and additives of anti-oxidants, pH adjusters and like did not influence the thermo-resistance, freeze-thaw resistance and particle size of the emulsions. The emulsions prepared by using the oil phase of C₆-C₂₈ oils selecting from one or more of structurally modified or hydrolyzed coconut oil, olive oil, soybean oil, safflower oil, triglycerides, octyl and decyl glycerate, ethyl oleate, glyceryl linoleate, ethyl linoleate, glyceryl oleate, cholesteryl oleate/linoleate, coconut oil C₈/C₁₀ monoglyceride or diglyceride, coconut oil C₈/C₁₀ propanediol diester, and coconut oil C₈/C₁₀ triglycerides, also obtained thermo-resistance and freeze-thaw resistance.

### Example 36: Stability experiments

### 1. Experiments on influencing factors

The experiments on high-temperature and illumination factors were designed and performed according to "Technical Guidelines in Chemical Drugs Stability", Chinese Pharmacopoeia, appendix II, 2005. Coenzyme Q₁₀ nano-emulsions were placed respectively under the conditions of 40°C and illumination (4500 ± 500 lux) for 10 days, and sampled at 5^{th} and 10^{th} days for detecting coenzyme Q₁₀ content and particle size distribution. The results are listed in Table 19.

**Table 19 Results of the experiments on influencing factors**

| Condition | Time (Day) | Appearance | Average particle size (nm) | CV | Content (%) |
|---|---|---|---|---|---|
| 40□ | 0 | Yellow emulsion | 71.3 | 0.399 | 102.2 |
| | 5 | Yellow emulsion | 66.6 | 0.407 | 101.2 |
| | 10 | Yellow emulsion | 64.9 | 0.437 | 101.6 |
| Illumination | 5 | Yellow emulsion | 68.5 | 0.411 | 54.8 |

It can be seen from above data, during the period of storing at 40°C, there was no significant change in coenzyme Q₁₀ content and average particle size for the nano-emulsions. Under illumination condition, the content decreased obviously at the 5^{th} day, and thus the illumination experiment was not performed on the 10^{th} day. There was no significant change in average article size in this illumination experiment. The results indicate the emulsions have a trend to be unstable under illumination condition, and thus should be stored in dark.

### 2. Accelerated experiment and long-term stability experiment

### (1) Accelerated experiment

The accelerated experiment was carried out at 25 ± 2°C for 6 months according to the "Technical Guidelines in Chemical Drugs Stability" in pharmacopoeia.

Coenzyme Q₁₀ emulsions were packaged in vials, filled with nitrogen, sealed and stored at 25 ± 2°C for 6 months in dark. The samples were taken at the 1^{st}, 2^{nd}, 3^{rd}, and 6^{th} months for detecting the changes in appearance, particle size and content The results are listed in Table 20.

**Table 20 Results of the accelerated experiment**

| Batch | Time (Month) | Appearance | Average particle size (nm) | CV | Content (%) |
|---|---|---|---|---|---|
| 1 | 0 | Yellow emulsion | 69.0 | 0.390 | 100.0 |
| | 1 | Yellow emulsion | 68.7 | 0.388 | 99.8 |
| | 2 | Yellow emulsion | 67.9 | 0.396 | 101.3 |
| | 3 | Yellow emulsion | 69.5 | 0.398 | 99.5 |
| | 6 | Yellow emulsion | 70.5 | 0.415 | 99.2 |
| 2 | 0 | Yellow emulsion | 66.8 | 0.401 | 100.0 |
| | 1 | Yellow emulsion | 68.9 | 0.389 | 99.5 |
| | 2 | Yellow emulsion | 69.6 | 0.400 | 100.8 |
| | 3 | Yellow emulsion | 71.8 | 0.413 | 99.04 |
| | 6 | Yellow emulsion | 70.6 | 0.421 | 100.1 |
| 3 | 0 | Yellow emulsion | 71.3 | 0.388 | 100.0 |
| | 1 | Yellow emulsion | 68.9 | 0.381 | 99.2 |
| | 2 | Yellow emulsion | 67.0 | 0.396 | 101.5 |
| | 3 | Yellow emulsion | 70.8 | 0.404 | 100.6 |
| | 6 | Yellow emulsion | 73.9 | 0.413 | 98.8 |

### (2) Long-term stability experiment

The long-term stability experiment was carried out at 6 ± 2°C according to the "Technical Guidelines in Chemical Drugs Stability" in pharmacopoeia.

Coenzyme Q₁₀ emulsions were packaged in vials, filled with nitrogen, sealed and stored at 6 ± 2°C for 6 months in dark. The samples were taken at the 3^{rd}, and 6^{th} months for detecting the changes in appearance, particle size and content The results are listed in Table 21.

**Table 21 Results of the long-term stability experiment**

| Batch | Time (Month) | Appearance | Average particle size (nm) | CV | Content (%) |
|---|---|---|---|---|---|
| 1 | 0 | Yellow emulsion | 69.0 | 0.390 | 100.0 |
| | 3 | Yellow emulsion | 70.2 | 0.396 | 101.5 |
| | 6 | Yellow emulsion | 67.5 | 0.405 | 99.2 |
| 2 | 0 | Yellow emulsion | 66.8 | 0.401 | 100.0 |
| | 3 | Yellow emulsion | 69.7 | 0.393 | 99.7 |
| | 6 | Yellow emulsion | 70.2 | 0.405 | 98.6 |
| 3 | 0 | Yellow emulsion | 71.3 | 0.388 | 100.0 |
| | 3 | Yellow emulsion | 69.7 | 0.392 | 99.2 |
| | 6 | Yellow emulsion | 66.6 | 0.399 | 99.8 |

The results show there is no obvious change in appearance, particle size and content for the coenzyme Q₁₀ emulsions of the present invention after storage under the accelerated conditions of 25 ± 2°C and long-term storage at 6 ± 2°C for 6 months in dark.

## Claims

1. A composite emulsfier, comprising two or more emulsifiers selected from a phospholipid ≤10%w/v, a PEG emulsifier ≤30%w/v, and a poloxamer≤10%w/v, calculated based on the emulsion.

2. The composite emulsifier according to claim 1, wherein the phospholipid is selected from one or more of yolk phospholipids, soybean phospholipids and other natural or semi-synthesized or synthesized phospholipids; the PEG emulsifier is selected from one or more of HS₁₅, TPGS and DSPE-PEG including DPPE-PEG and DMPE-PEG, wherein the molecular weight of PEG is 100-10000; and the poloxamer is selected from the group consisting of poloxamer 188 and Pluronic F68.

3. The composite emulsifier according to claim 2, wherein the other natural or semi-synthesized or synthesized phospholipids are selected from the group consisting of cardiolipin, phosphatidylinositol, phosphatidylglycerol, sphingomyelin, phosphatidylserine, hydrogenated lecithin, dipalmitoyl phosphatidyl choline, dioleoyl phosphatidyl choline, dilauroyl phosphatidylcholine, and phosphatidylethanolamine.

4. The composite emulsifier according to claim 1, wherein the composite emulsifier comprises a phospholipid, a PEG emulsifier and a cryoprotectant, wherein the cryoprotectant is in an amount of ≤50%w/v, calculated based on the emulsion.

5. The composite emulsifier according to claim 4, wherein the cryoprotectant is selected from the group consisting of an alcohol and a saccharide, in which the alcohol is selected from one or more of propanediol, glycerol and polyethylene glycol, and the saccharide is selected from one or more of glucose, mannitol, sucrose, trehalose, maltose and lactose.

6. The composite emulsifier according to claim 1, wherein the composite emulsifier comprises three or more emulsifiers, one of which is a poloxamer, but not comprises a cryoprotectant.

7. The composite emulsifier according to claim 6, wherein the composite emulsifier comprises a phospholipid and/or a PEG emulsifier and a poloxamer emulsifier.

8. An emulsion prepared with the composite emulsifier of claim 1, comprising an oil phase, the composite emulsifier and a water phase, wherein the composite emulsifier is selected from two or more of the following: a phospholipid ≤10%w/v, a PEG emulsifier ≤30%w/v, and a poloxamer ≤10%w/v, calculated based on the emulsion.

9. The emulsion according to claim 8, wherein the oil phase comprises a C₆-C₂₈ oil, an oily material with therapeutic activity and/or a lipophilic compound or drug dissolved or dispersed in the C₆-C₂₈ oil; in which the weight ratio of the lipophilic compound or drug to the C₆-C₂₈ oil is 1:0 to 1:10000 w/w, and the amount of the C₆-C₂₈ oil is 0.1-20% w/v, calculated based on the emulsion.

10. The emulsion according to claim 8, wherein the C₆-C₂₈ oil is selected from one or more of structurally modified or hydrolyzed coconut oil, olive oil, soybean oil, safflower oil, triglycerides, octyl and decyl glycerate, ethyl oleate, glyceryl linoleate, ethyl linoleate, glyceryl oleate, cholesteryl oleate/linoleate, coconut oil C₈/C₁₀ monoglyceride or diglyceride, coconut oil C₈/C₁₀ propanediol diester, and coconut oil C₈/C₁₀ triglycerides; the oily material with therapeutic activity and/or the lipophilic compound or drug dissolved or dispersed in the C₆-C₂₈ oil is selected from coenzyme Q₁₀, cucurbitacin, cucurbitacin B, dihydrocucurbitacin B, isocucurbitacin B, cucurbitacin D, cucurbitacin E, cucurbitacin I, cucurbitacin Q, alprostadil, diisopropylphenol, vitamin K1, dexamethasone palmitate, tanshinone IIA, butylphthalide, ligustilide, irisquinone, entecavir, anethol trithione, malotilate, homoharringtonine, demethylcantharidate, curcumine, cyclandelate, β-elemene, batyl alcohol, statins hypolipidemic drugs, *Brucea javanica* oil, sea buckthorn oil, fish oil, coix seed oil , zedoary turmeric oil, garlic oil, allicin, *Chuanxiong* rhizome oil, angelica oil, capsicum oil, ginger oil, celery seed oil, *Houttuynia cordata* oil, Evening primrose oil, perilla seed oil, Wufu Xinnaokang, tocopherol nicotinate, gossypol, oridonin, fenofibrate, itraconazole, candesartan, hydroxycamptothecin, camptothecin derivatives, paclitaxel and the derivatives thereof, docetaxel and the derivatives thereof, finasteride, etoposide, ginsenoside, 20(S)-protopanaxadiol, ginsenoside Re, ginsenoside Rb1, ginsenoside Rg₂, 20(R)-ginsenoside Rg₃ and 20(S)-ginsenoside Rg₃.

11. The emulsion according to claim 8, wherein the emulsion further comprises one or more pharmaceutical excipients selected from a co-emulsifier, a stabilizer, a cryoprotectant and a pH adjuster; in which the co-emulsifier is selected from one or more of oleic acid, linoleic oil, linolenic acid, stearic acid, docosahexaenoic acid and cholic acid; the cryoprotectant is selected from one or more of an alcohol including propanediol, glycerol and polyethylene glycol, and one or more of saccharides including glucose, mannitol, sucrose, trehalose, xylitol, maltose and lactose; the stabilizer is selected from one or more of nitrogen, EDTA and salts thereof, anhydrous sodium sulfite, anhydrous sodium hydrogen sulfite, sodium pyrosulfite, vitamin C and derivatives thereof, butylated hydroxytoluene, α-tocopherol, α-tocopherol acetate and hydroquinone; the isoosmotic adjustment agent is selected from one or more of glycerol, 1,2-propanediol, glucose, maltose, mannitol and xylito; and the pH adjuster is selected from one or more of hydrochloric acid, sodium hydroxide, acetic acid, sodium acetate, phosphoric acid, sodium phosphate, citric acid and sodium citrate.

12. The emulsion according to claim 8, wherein the water phase is water and/or glycerol-water solution with or without a water-soluble substance.

13. A method for preparing the emulsion according to claim 8, comprising the following steps:
1) preparing the oil phase: heating the oil and lipophilic material to 20-90°C under nitrogen atmosphere, then adding the emulsifier, stirring at 20-90°C till dissolved, and adding an active ingredient under stirring;
2) preparing the water phase: adding the water-soluble material into water and/or emulsifier used in water phase, and stirring at 20-90°C for 5 min till completely mixed and dissolved;
3) adding the water phase into the oil phase under nitrogen atmosphere at 20-60°C, and stirring for 5-30 min to obtain the primary emulsion;
4) homogenizing with a homogenizer firstly under a pressure of 4000-8000 psi and then under a pressure of 10000-30000 psi to obtain the emulsion.

14. The method according to claim 12, wherein the pH value is adjusted to 3-9 before homogenization.

15. The method according to claim 12, wherein the method further comprises the steps of filtration with microporous membrane, package and sterilization.

16. The method according to claim 14, wherein the sterilization is selected from the group consisting of aseptic processing, moist heat sterilization, autoclave sterilization and microwave sterilization.
